(19) Europäisches Patentamt / European Patent Office / Office européen des brevets

(11) **EP 3 253 730 B1**

(12) **EUROPÄISCHE PATENTSCHRIFT**

(45) Veröffentlichungstag und Bekanntmachung des Hinweises auf die Patenterteilung:
**17.04.2019 Patentblatt 2019/16**

(21) Anmeldenummer: **16719194.9**

(22) Anmeldetag: **04.02.2016**

(51) Int Cl.:
*C07C 51/47* (2006.01)    *C07C 55/02* (2006.01)
*C07C 55/10* (2006.01)    *C07C 57/13* (2006.01)
*C07C 57/145* (2006.01)    *C07C 57/155* (2006.01)
*C07C 59/245* (2006.01)    *C07C 59/255* (2006.01)

(86) Internationale Anmeldenummer:
**PCT/DE2016/000038**

(87) Internationale Veröffentlichungsnummer:
**WO 2016/124170 (11.08.2016 Gazette 2016/32)**

(54) **VERFAHREN ZUR TRENNUNG ORGANISCHER DICARBONSÄUREN DURCH ADSORPTION AN HYDROPHOBEN PORÖSEN MATERIALIEN**

METHOD FOR SEPARATING ORGANIC DICARBOXLIC ACIDS BY ADSORPTION ON HYDROPHOBIC POROUS MATERIALS

PROCÉDÉ DE SÉPARATION D'ACIDES DICARBOXYLIQUES ORGANIQUES PAR ADSORPTION SUR MATÉRIAUX POREUX HYDROPHOBES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **06.02.2015 DE 102015001407**
               **02.02.2016 DE 102016001070**

(43) Veröffentlichungstag der Anmeldung:
**13.12.2017 Patentblatt 2017/50**

(73) Patentinhaber: **RWTH Aachen**
**52062 Aachen (DE)**

(72) Erfinder:
• **PALKOWITZ, Regina**
**52074 Aachen (DE)**
• **ROSE, Marcus**
**52074 Aachen (DE)**
• **SCHUTE, Kai**
**52064 Aachen (DE)**

(74) Vertreter: **Sparing, Rolf Klaus et al**
**Bonnekamp & Sparing**
**Patentanwaltskanzlei,**
**European Patent & Trade Mark Law Firm,**
**Goltsteinstrasse 19**
**40211 Düsseldorf (DE)**

(56) Entgegenhaltungen:
**WO-A1-2013/169447**

• **CHIA-YUAN C. LEE ET AL: "Adsorption of oxalic, malonic, and succinic acids on activated carbon", JOURNAL OF CHEMICAL AND ENGINEERING DATA., Bd. 31, Nr. 2, 1. April 1986 (1986-04-01), Seiten 133-136, XP055281306, US ISSN: 0021-9568, DOI: 10.1021/je00044a001**
• **CHALINE DETONI ET AL: "Selective Liquid Phase Adsorption of 5-Hydroxymethylfurfural on Nanoporous Hyper-Cross-Linked Polymers", ACS SUSTAINABLE CHEMISTRY & ENGINEERING, Bd. 2, Nr. 10, 19. August 2014 (2014-08-19), Seiten 2407-2415, XP055280476, US ISSN: 2168-0485, DOI: 10.1021/sc5004264**

EP 3 253 730 B1

**Beschreibung**

Die Erfindung betrifft ein Verfahren zur Abtrennung mehrerer Dicarbonsäuren oder Fraktionen mehrerer Dicarbonsäuren aus biologischen und/oder chemischen Reaktionslösungen durch Adsorption bei einem pH-Wert 1 bis 7 in einem Temperaturbereich von 20°-80°C an zumindest teilweise unpolare, hydrophobe Adsorbention, von denen nachfolgend ein Adsorbat desorbiert wird.

Die Stofftrennung fester Stoffe in Lösungen ist besonders für fermentative Prozesse mit niedrigen Umsätzen oder bei Prozessen mit Erzeugung von Nebenprodukten von großer Bedeutung und wird im Rahmen einer wachsenden Biotechnologie-Branche noch weiter an Bedeutung zunehmen. Verfahren zur Flüssigphasenadsorption bieten dabei ein großes Potential für derartige Anwendungen, vorausgesetzt die verwendeten Adsorbentien bieten die gewünschten Eigenschaften hinsichtlich Kapazität und Selektivität. Im Falle von fermentativen Prozessen etwa liegen neben nicht umgesetztem Substrat oftmals Produktgemische unterschiedlich polarer Komponenten wie z.B. Dicarbonsäuren vor.

Die Stofftrennung durch Flüssigphasenadsorption in biotechnologischen Prozessen gewinnt im Bereich der Forschung und Entwicklung immer mehr an Bedeutung. Der Bedarf entsteht durch die Entwicklung und Kommerzialisierung bio-basierter Plattformchemikalien und neuer Produkte und dem dafür notwendigen Einsatz effizienter Produktionsverfahren. Ein prominentes Beispiel dafür ist Itaconsäure, die bereits mit einer Jahreskapazität von ca. 80 kt hergestellt wird. Die fermentativen Verfahren werden bei Temperaturen unter 40 °C in wässrigen Lösungen durchgeführt. Optimierte Verfahren erreichen heutzutage Produktkonzentrationen von max. 86 g/L. Der Grund dafür ist die typischerweise auftretende Substrat- und v. a. Produktinhibierung. Die Abtrennung der Produkte erfolgt in zwei Schritten, die verschiedene Verfahrensschritte verbinden. Zuerst wird die Produktlösung von Feststoffen aller Art durch Filtration bzw. in effizienteren Verfahren durch membranbasierte Mikrofiltration getrennt. Danach wird durch Kristallisation technisch reine Itaconsäure gewonnen. Um die Abtrennung effizienter zu gestalten, eine kontinuierliche Prozessführung mit in situ-Produktabtrennung zu ermöglichen sowie die gesamte Produktivität zu verbessern, sollte das entstehende Produkt während des Fermentationsschrittes kontinuierlich abgetrennt werden. Adsorption aus der wässrigen Lösung könnte daher ein vielversprechender Ansatz sein. Die Adsorption von Dicarbonsäuren in Form von Itaconsäure wurde bisher lediglich an Aluminiumoxid untersucht, das durch eine sehr geringe spezifische Oberfläche vergleichsweise schlechte Adsorptionseigenschaften aufweist.

Neben Itaconsäure werden jedoch eine ganze Reihe weiterer C4- und C5-Dicarbonsäuren, wie z. B. Bernsteinsäure, Äpfelsäure und Fumarsäure, ebenfalls fermentativ hergestellt. Auch können diese als Koppelprodukte in Fermentationsschritten auftauchen, was zu einem zusätzlichen Prozessaufwand führt, um die entsprechenden Reinstoffe zu trennen. So ist aus dem Stand der Technik bekannt, dass für die Adsorption von Bernsteinsäure Hydrotalcite, aluminiumarme hydrophobe Zeolithe sowie basisch funktionalisierte lonentauscherharze vielversprechende Eigenschaften aufweisen, wobei die Adsorption auf Säure-Base- bzw. ionischen Wechselwirkungen beruhen. Verschiedene basische lonentauscherharze bzw. aminiertes, poröses Chitosan sind für die Trennung von Fumarsäure und Äpfelsäure bislang untersucht worden.

So ist aus der DE 10 2011 120 632 A1 ein Verfahren zur Abtrennung und Aufreinigung von Carbonsäuren aus Fermentationsbrühen bekannt, wobei das Verfahren nach Abtrennung der Feststoffe und Reinigung der Fermentationsbrühen, eine Abtrennung der Carbonsäuren, insbesondere Itaconsäure an eine oder mehrere feste Phasen erfolgt, die tertiäre Aminogruppen aufweisen.

Ferner ist in der EP 0 377 430 A1 ein Verfahren zur Abtrennung von Säuren aus salz-und kohlehydrathaltigen Substraten offenbart, bei denen die Substrate über Harze geleitet werden, die die Säuren adsorptiv aus dem Substrat entfernen, und anschließend die Säuren durch Elution von den Harzen abgetrennt werden, mit der Besonderheit, dass als Harze basische lonentauscherharze eingesetzt werden.

In der Druckschrift US 2,697,725 A ist ein Verfahren zum Erhalt organischer Säuren aus Fermentationsprozessen beschrieben, bei dem mittels ionischer Austauschharze und mittels geeigneter pH-Wahl Carbonsäuren, u.a. auch Itaconsäure gewonnen werden kann.

Ferner ist aus der WO 2013/169447 A1 ein Verfahren zur Abtrennung von Bernsteinsäure aus einer Fermentationsbrühe durch Filtrieren. Ansäuern des Filtrats und säulenchromatographische Trennung des Filtrats an einem nichtfunktionalisiertem Harz beschrieben.

In der Druckschrift Chia-Yuan C. Lee et al.: Adsorption of oxalic, malonic and succinic acids on activated carbon" in Journal of chemical and engineering data. Bd. 31, Nr.2, 1. April 1986 ist offenbart, dass Aktivkohle zur Adsorption von Bernsteinsäure generell geeignet ist.

Schließlich ist in der Druckschrift Chaline Dtoni et al.: Selctive liquid phase adsorption of 5-hydroxymethylfurfural on nanopourous hyper-cross-linked Polymers, in ACS sustainable chemistry & engineering. Bd. 2, Nr. 10, 19. August 2014 eine Verwendung von HCP zur Adsorption u.a. von Lävulinsäure. Beschrieben.

In dem Stand der Technik erfolgt jedoch stets nur die Adsorption einer ausgewählten Komponente bzw. die Trennung einer Carbonsäure und seines Substrates, wobei die Adsorption und/oder die nachfolgende Elution Probleme aufwerfen. Zudem ist eine selektive Trennung von mehreren in Lösung oder allgemein in Fermentationsbrühen koexistierenden Dicarbonsäuren bislang nicht beschrieben und kann mit konventionellen Adsorbentien auch nicht erreicht werden.

Aufgabe der Erfindung ist es eine Adsorption von Dicarbonsäuren und eine direkte Trennung koexistierender Dicarbonsäuren unterschiedlicher Polarität an hydrophobe Materialien bzw. Adsorbentien mittels Flüssigphasenadsorption mit hoher Selektivität zu gewährleisten.

Die Aufgabe wird durch die Merkmale des unabhängigen Anspruchs 1 gelöst. Die Erfindung, für die Schutz beansprucht wird, bezieht sich nur auf diejenigen in der Beschreibung erläuterten Ausführungsform, die unter den unabhängigen Anspruch 1 fallen.

Zur Trennung von Produktgemischen eignen sich insbesondere hoch hydrophobe Materialien. Für hydrophile Materialien existiert zwar eine etablierte Standardmethode, um die Hydrophobie quantitativ beschreiben zu können. Eine Methode, die oft eingesetzt wird, um unterschiedliche Materialien in Relation zueinander zu beschreiben, ist im Fall hydrophober Materialien die Adsorption von Wasserdampf. Wasser ist eine stark polare Verbindung und zeigt nur schwache Wechselwirkungen und entsprechend eine geringe Affinität für die Adsorption an hydrophoben Oberflächen. Dementsprechend kann über das Verhalten eines porösen Materials in der Wasserdampfsorption auf den hydrophoben Charakter der Oberfläche geschlossen werden. Durch die Form der Isotherme und den Vergleich der bei bestimmten Relativdrücken adsorbierten Menge Wasser mit dem gesamt verfügbaren Porenvolumen, das aus der Adsorption von Stickstoff bestimmt wird, können verschiedene Materialien semi-quantitativ miteinander verglichen werden.

Die Adsorption von Stickstoff ist eine standardisierte Methode und wird bei -196°C durchgeführt. Die wichtigste daraus bestimmte Größe ist das totale Porenvolumen. Wasserdampfsorption ist nicht standardisiert. Typischerweise werden die Messungen bei 20-50°C durchgeführt. Für die Vergleichbarkeit einzelner Materialien ist die gleiche Messtemperatur notwendig. Im Folgenden sind aus Publikationen der Arbeitsgruppe zwei Beispiele gezeigt, wie der hydrophobe Charakter der HCPs mit der Synthese variiert bzw. auch der Unterschied zu einer Aktivkohle und einem Zeolithen.

Als Adsobentien können Aktivkohlen wie A Supra A EUR, Darco G 60, SxIG, RX3 und Norti, weiterhin hochvernetzte Polymere (High Crosslinked Polymers - HCP), insbesondere vernetzte Polystyrole und/oder Davankov-Harze eingesetzt werden. Die HCP's können insbesondere Polymethylenbenzol und Polymethylenbiphenyl umfassen.

Erfindungsgemäss werden als Adsorbentien HCP eingesetzt, welche ausgewählt sind aus Polymethylenbiphenyl und Polymethylenbenzol.

Es wurde im Rahmen der vorliegenden Offenbarung ein Molekülportfolio unterschiedlicher Dicarbonsäuren untersucht, welche insbesondere in fermentativen Prozessen hergestellt werden. Als vielversprechende Adsorbentien mit hoher Selektivität wurden hydrophobe Aktivkohlen und Polymere, wie beispielsweise HCP's identifiziert.

Die beschriebenen Adsorptionsmittel unterscheiden sich in der maximalen Aufnahmefähigkeit von Glucose. So sind Aktivkohlen, wie beispielsweise A Supra EUR in der Lage, größere Mengen Glucose als die HCP's an der Oberfläche zu adsorbieren, was auf die Anwesenheit von polaren funktionellen Gruppen auf der Oberfläche der Aktivkohle zurückzuführen ist und auf eine stärkere Affinität der Oberfläche der Aktivkohle gegenüber zu Glucose schließen lässt.

Die Adsorption aus einer flüssigen Phase hängt von den Wechselwirkungen zwischen Adsorbens und Adsorbat, Adsorbat-Adsorbat, Lösungsmittel-Adsorbens und Lösungsmittel-Adsorbat ab, was zu einem komplexen Adsorptionssystem führt. Die Adsorptionskapazitäten der Adsorbentien verringern sich jedoch in Mehrkomponenten-Lösungs-Systemen im Vergleich zu den Einzelkomponenten-Lösungs-Systemen, die mit einer konkurrierenden Adsorption der Reagenzien an den Oberflächen der Adsorbentien zusammenhängt. Die Hydrophobie der aromatischen Struktur der HCP führt zu einer stark selektiven Adsorption und einer hohen Adsorption von Itaconsäure, wobei eine niedrige Affinität zu Glucose feststellbar ist. Demgegenüber zeigen die Aktivkohle-Adsorbentien, wie beispielsweise .A Supra EUR und basische Ionentauscherharze. Harze wie Amberlite ein schwächeres Absorptions- und auch Desorptionsverhalten in Bezug auf die Gesamtmenge des adsorbierten Di-Carbonsäure Reaktionsprodukts. A Supra EUR zeigt eine höhere Affinität zu Glucose als die HCP's, was auf die hydrophilen Gruppen (sauerstoffhaltigen Gruppen) zurückzuführen sein könnte und offenbart daher eine geringere Adsorptionskapazität von Dicarbonsäuren im Vergleich zu HCP's. Als Ergebnis kann damit festgestellt werden, dass der Anteil der Adsorption von Glucose gegenüber einer Dicarbonsäure, beispielsweise Itaconsäure bei unpolaren, hydrophoben Adsorbentien geringer ist und umgekehrt der Anteil der an den Adsorbentien anhaftenden Dicarbonsäure bei polaren hydrophilen Adsorbentien höher ist. Dies hat Auswirkungen darauf, dass durch Zusatz geeigneter, hydrophiler Adsorbentien ein großer Anteil bio-technologisch hergestellter Dicarbonsäuren aus Fermentationsprozessen herausgezogen und anschließend leicht durch Elution gewonnen werden kann. Die Zunahme des Grads der Hydrophobie der Adsorbentien HCP > A Supra EUR > Amberlite führt zu einer höheren Adsorptionskapazität in Bezug auf die Dicarbonsäuren wie beispielsweise Itaconsäure. Umgekehrt ist bei den Oberflächen der vorgenannten Adsorbentien eine anschließende Desorption der Itaconsäure durch polare Lösungsmittel wie Wasser und/oder Alkohole erleichtert, d.h. dass eine größere Desorption von Itaconsäure mit der Zunahme des Grads der Hydrophobie der Adsorbentien erfolgt und zwar in der Reihenfolge Amberlite < A Supra EUR < HCP. Bei der kompetitiven Adsorption, zeigt sich dieser Effekt auch durch die Wechselwirkung und die Adsorption von Wasser auf der Oberfläche.

So sind auch durch Messungen von Wasserdampf an HCP Isothermen bestimmbar, die zeigen, dass bei einer Adsorption weniger als 80% des freien Porenvolumens mit Wasserdampf bei einer relativen Feuchte von über 90% belegt sind. Grund dafür ist die Hydrophobie der Oberfläche von HCP, wobei erfindungsgemäß als Hydrophobie eine Aufnahmekapazität der Porenvolumen der Oberflächen der Adsorbentien von weniger als 80% bei einer relativen Feuchte von 90% definiert ist. Allgemein wurde ermittelt, dass die Zunahme des Grads der Hydrophobie der Adsorbentien zu einer höheren Adsorptionskapazität in Bezug auf eine Dicarbonsäure führt. Umgekehrt wurde bei den Oberflächen der vorgenannten Adsorbentien gefunden, dass eine anschließende Desorption der Itaconsäure an den zumindest teilweise unpolaren Oberlächen durch polare Lösungsmittel wie Wasser und/oder Alkohole erleichtert ist, d.h. das eine größere Desorption von Dicarbonsäuren mit der Zunahme des Grads der Hydrophobie der Adsorbentien erfolgt. Einige Adsorbentien wie Aktivkohlen zeigen zwar ähnliche Adsorptionskapazitäten wie HCP's. Allerdings unterscheidet sich das Desorptionsverhalten von einigen Aktivkohlen gegenüber denen wie HCP und zwar in dem Maße wie der Anteil polarer, hydrophiler Gruppen auf der Oberfläche zunimmt. So wurde gefunden, dass bei Chemiesorption an der Oberfläche bzw. durch Säure-Base-Wechselwirkungen aufgrund der funktionellen sauerstoffhaltigen Gruppen die Adsorption von Dicarbonsäuren in Gegenwart von Glucose geringer ist. Durch die Art des Adsorptionsvorgangs und der anschließenden Desorption in Gegenwart von polaren oder sauerstoffhaltigen Gruppen ist die Desorption der Reaktionsprodukte von der Oberfläche bei angenäherter Chemiesorption erschwert. Durch die Verwendung zumindest teilweise unpolarer Adsorbentien wie HCP, wurde gefunden, dass es möglich ist, nahezu 100% der adsorbierten Itaconsäure von den Adsorbentien zu erhalten.

Die beschriebenen Adsorbentien weisen demnach zumindest teilweise eine Hydrophobie auf und haben eine zumindest teilweise unpolare Oberfläche, wobei das Porenvolumen der Adsorbentien eine Größe von 0,01 cm$^3$/g bis 4,0 cm$^3$/g und die Adsorbentien eine Oberfläche im Bereich von 100 m$^2$/g bis 4000 m$^2$/g umfassen. Die Art der verwendeten Adsorbentien entspricht den im beschriebenen Verfahren eingesetzten Stoffe und Verbindungen, wie HCP's, Aktivkohle und Davankov-Harze.

Für die Desorption der an den Adsorbentien anhaftenden bzw. adsorbierten Dicarbonsäuren können unterschiedliche Lösungsmittel verwendet werden. Dabei ist Wasser ein Lösungsmittel, das die Desorption ermöglicht. Ferner sind auch wässrige Lösungen oder organische Lösungsmittel auf Basis von Aldehyden, Ketonen, Aminen, Carbonsäuren und/oder Alkoholen oder deren Gemische verwendbar, insbesondere können Alkohole wie Methanol oder Ethanol sowie Aldehyde wie Aceton verwendet werden. Die Wahl der Lösungsmittel richtet sich nach den Reaktionsbedingungen, der Löslichkeit von Dicarbonsäuren in diesen und den Kosten für die Bereitstellung der Lösungsmittel. Die Desorption erfolgt in der Regel bei einem pH-Wert im Bereich größer oder gleich 7.

Ein weiteres wichtiges Merkmal für die Wahl des entsprechenden Desorptions-Lösungsmittels ist die Fähigkeit, Wechselwirkungen mit dem Adsorbat auszubilden, da die Affinität zwischen den beiden Carboxylgruppen und dem Lösungsmittel stark genug sein müssen, um die Desorption zu begünstigen. Die Adsorption an den Adsorbentien erfolgt in Abhängigkeit vom gewählten Adsorbens bzw. den Eigenschaften der Adsorbentien, die zwar geringe aber trotzdem bestimmbare Unterschiede in der Oberflächenstruktur aufweisen. So können poröse Kohlenstoffmaterialien, insbesondere Aktivkohle mehr oder stärkere ionische Wechselwirkungen zur Itaconsäure ausbilden als poröse Polymere, insbesondere HCP und Davankov-Harze, die über Wasserstoffbrückenbindungen und van-der-Waalsbindungen eine Wechselwirkung mit den Oberflächen der Adsorbentien ausbilden.

Es wurde in Folge der unterschiedlichen Wechselwirkungen erfindungsgemäß gefunden, dass die Desorption am effizientesten bei HCP erfolgt, da fast die Gesamtmenge adsorbierter Dicarbonsäuren unter Verwendung von Wasser als Lösungsmittel desorbiert werden kann. Alkohole wie Methanol und Ethanol, wie auch beispielsweise Aceton, begünstigen ebenfalls die Desorption der Dicarbonsäuren an HCP. Aktivkohlen wie AC50 und basischen Ionentauscherharze wie Amberlite zeigen demgegenüber aber ein schwächeres Desorptionsverhalten von weniger als 30% Freisetzung von Itaconsäure in Bezug auf die Gesamtmenge des adsorbierten Reaktionsprodukts. Grund dafür sind die stärkeren ionischen Wechselwirkungen zwischen den Oberflächen dieser Adsorbentien und den Dicarbonsäuren.

Für co-existierende Dicarbonsäuren wurden signifikante Unterschiede für die Adsorption hinsichtlich der Polarität beobachtet und durch Berechnung des Dipolmoments quantifiziert. Adsorptionsstudien im Satzbetrieb sowie in kontinuierlicher Betriebsweise wurden zur Bestimmung von Ein- und Mehrkomponentenisothermen sowie Durchbruchskurven durchgeführt. Co-existierende Carbonsäuren bedeutet vorliegend, dass innerhalb eines oder mehrerer nachgeschalteter Fermentationsprozesse simultan verschiedene Dicarbonsäuren vorhanden sein können, die unterschiedlich stark an die eingesetzten Adsorbentien adsorbieren können. Für die Adsorbentien wurde bei allen untersuchten Säuren eine bevorzugte Adsorption der jeweils unpolareren Säure beobachtet. Die Adsorptionsselektivität nimmt dabei mit steigender Polaritätsdifferenz zwischen den Säuren zu, wohingegen die Adsorptionskapazität für die Adsorbentien nahezu unverändert bleibt. Mit dieser unerwarteten Selektivität in der co-Adsorption mehrerer Dicarbonsäuren aus Gemischen bietet die Flüssigphasenadsorption großes Potential als alternative Trennmethode in Biorafinerieprozessen. Wird das Adsorptionsverhaltens biomassebasierter Dicarbonsäuren unterschiedlicher Polarität durch Einkomponenten- sowie Mehrkomponentenadsorptionen, also in Gegenwart einzelner oder mehrerer Dicarbonsäuren untersucht, so stellt sich nach vorhergegangenem Adsorbensscreening heraus, dass poröse unpolare Adsorbentien für eine hohe Adsorptionskapazität als auch Selektivität entscheidend sind. Als geeignete Adsorbentien mit hohen spezifischen Oberflächen und stark hydrophoben Eigenschaften, wurden ein hochvernetztes, hydrophobes Polymer (HCP) sowie zum Vergleich verschiedene kommerziell verfügbare Materialien untersucht. Ähnlich gute Adsorptionseigenschaften wie das HCP zeigten hierbei z.B. polymerbasierte sphärische Aktivkohlen (PBSAC/AC50), die sich wie die Polymere durch einen sehr geringen Grad an Oberflächenfunktionalisierung auszeichnen.

Es wurde festgestellt, dass je hydrophober ein poröses Material ist, desto höher die Selektivität für die Adsorption ist.

Die Konkurrenzadsorption verschiedener Dicarbonsäuren und ihrem Substrat wie beispielsweise Glucose ist an porösen Polymeren wie HCP, insbesondere (Polymethylenbiphenyl) als hydrophobes Adsorbens bevorzugt. Hydrophobe, unpolare Adsorbentien adsorbieren Dicarbonsäuren in signifikant höheren Mengen als solche mit weniger hydrophoben, unpolaren Oberflächen. So wird an hydrophoben, unpolaren Oberflächen die polare Glucose über einen breiten Konzentrationsbereich gar nicht adsorbiert. Die höchste Selektivität für eine Dicarbonsäureadsorption, insbesondere Itaconsäureadsorption ist gegenüber Glucose mit HCP-Adsorbentien erzielbar, was auf die hohe Hydrophobie des HCP aufgrund seiner unpolaren organischen Netzwerkstruktur zurückzuführen ist.

Die totale Adsorption wird primär dadurch bestimmt, wie hoch die verfügbare spezifische Oberfläche der Adsorbentien ist. Damit ein Material hydrophob genug ist, um eine hohe Selektivität für die Adsorption aufzuweisen, sollte keine signifikante Adsorption von Wasser bei Relativdrücken ($p/p_0$) kleiner 0,6 stattfinden. Im Relativdruckbereich 0,8-1,0 sollte eine maximale Füllung der Poren vorliegen, die geringer ist, als das total verfügbare Porenvolumen, das aus der Stickstoffsorption bestimmt wurde.

Idealerweise besitzen die Adsorbentien ein Verhalten wie die Polymethylenbiphenyl-HCPs und adsorbieren bei $p/p_0<0,8$ gar kein Wasser und der Porenfüllungsgrad, also der Anteil an mit Wasser gefülltem Porenvolumen, ist bei $p/p_0=0,9$ kleiner als 50%.

Für die Durchführung des beschriebenen Verfahrens wurde zunächst die Einkomponentenadsorption verschiedener Dicarbonsäuren und Glucose als dem wichtigsten Substrat für die fermentative Herstellung an AC50 und HCP untersucht. Die Produkte umfassten insbesondere $C_4$- und $C_5$- Dicarbonsäuren, wie Malein-, Itacon-, Bernstein-, Mesacon-, Citracon-, Methylbernstein-, Wein- und Methylbernsteinsäure. Des Weiteren wurden sämtliche Dipolmomente der Säuren mittels DFT-Berechnungen bestimmt. Zwischen den Komponenten ist ein auffälliger Polaritätsunterschied (Dipolunterschied) zu beobachten, welcher als einer der wichtigsten Triebkräfte für die Adsorption an unpolaren, hydrophoben Oberflächen darstellt.

Es wurde herausgefunden, dass die Polarität der Adsorbens einen entscheidenden Einfluss auf das Adsorptionsverhalten an hydrophoben Adsorbentien aufweist. Dies gilt auch für binäre Dicarbonsäuremischungen, sodass selektiv nach der Polarität aufgeschlüsselt werden kann zu adsorbieren. Dies ist von besonderem Interesse für Fermentationsprozesse, in denen neben dem gewünschten Zielprodukt weitere Produkte/Säuren anderer Polarität anfallen.

So konnte an ausgewählten Adsorbentien in äquimolaren, binären Dicarbonsäuremischungen gezeigt werden, dass die Säure mit geringerer Polarität bevorzugt adsorbiert wird. Wobei die Ausprägung der Selektivität mit steigendem Polaritätsunterschied zunimmt.

Steigt die Polaritätsdifferenz, so wird die Säure mit geringerer Polarität bevorzugt adsorbiert, wobei die Ausprägung der Selektivität mit steigendem Polaritätsunterschied zunimmt. Im System Itaconsäure/Bernsteinsäure adsorbieren beide Säuren nahezu gleichermaßen stark, während im Fall Itaconsäure/Äpfelsäure nahezu ausschließlich Itaconsäure adsorbiert wird. Im Falle der Konkurrenzadsorption von Bernsteinsäure und Maleinsäure deren Polaritätsdifferenz sehr gering ist, kann ebenfalls eine Selektivität hin zur unpolaren Substanz Bernsteinsäure beobachtet werden.

Im Falle des HCP ist dieser Trend wesentlich ausgeprägter. Schon in der Co-Adsorption von Itacon- und Bernsteinsäure zeichnet sich eine thermodynamisch bevorzugte Trennung von Bernsteinsäure ab, die bei Erhöhung der Konzentration noch deutlicher zunimmt. Für Malein- oder Äpfelsäure verstärkt sich der Trend der Adsorptionspräferenz hin zur Itaconsäure an HCP weiter. Auch für die Adsorptionsuntersuchung von Bernstein- und Maleinsäure reichert sich Bernsteinsäure bevorzugt an der Oberfläche des HCP an. Verglichen mit der AC50 kann eine deutliche Steigerung der Trennleistung erreicht werden.

Wichtig für die Durchführung des beschriebenen Verfahrens waren Untersuchungen, die insbesondere die Transporteigenschaften der Adsorbentien betreffen. Dafür wurden kontinuierliche Messungen durchgeführt. Die daraus resultierenden Durchbruchskurven ermöglichen neben Kapazitätsbestimmungen des Festbetts, kinetische Aussagen der Adsorption zu treffen.

Es wurden vier unterschiedliche Säuren an AC50 untersucht und die Durchbruchskurven ermittelt. Auffallend ist zunächst, dass in allen Experimenten ein klar definierter verzögerter Durchbruch zu beobachten ist. Für Itaconsäure wurde ein definierter Durchbruch ab 25 min erhalten, während für Äpfelsäure der Durchbruch bei 12 min erreicht ist. Bei Betrachtung der Gesamtkapazität $q_{th}$ ist der gleiche, polaritätsabhängige Trend zu beobachten wie auch in den Batchversuchen (Tabelle 1). Dies führt zu einer vergleichbaren Kapazität, wie aus den Isothermen hervorgeht. Bei Betrachtung der kinetischen Daten $k_{th}$ fällt insbesondere auf, das der Stofftransport gegenläufig ist. Mit steigender Polarität ist eine Zunahme des Transports zu erkennen. Der Stofftransport der Äpfelsäure wird hierdurch nahezu verdoppelt, verglichen mit Itaconsäure. Mögliche Ursache mag hierfür eine eventuell schlechtere Benetzung der unpolaren Säuren am Adsorbens sein, wodurch die Oberflächenadsorption schlicht gehindert oder verzögert wird. Die polaren Säuren wie z.B. Äpfelsäure benetzen die Oberfläche deutlich besser, obgleich die Gesamtkapazität deutlich geringer ist, und können hierdurch schneller die zugänglichen freien Adsorptionsplätze besetzen, weshalb die Transportkinetik begünstigt ist.

**Tabelle 1.:** Daten der Durchbruchskurven. Mit $c_0$ als Ausgangskonzentration, m als Masse des Adsorbens sowie $K_{th}$ als Kinetikkonstante und $q_{th}$ als Gesamtkapazität erhalten aus dem Thomas Modell.

| Säure | $c_0$ [mg/mL] | m [g] | $q_{th}$ [mg/g] | $K_{th}$ [mL/mg *min] | $q_{th}$ [mmol/g] | $K_{th}$ [mL/ mmol *min] |
|---|---|---|---|---|---|---|
| Äpfelsäure | 18,87 | 0,7835 | 281,0 | 0,064 | 2,096 | 8,502 |
| Bernsteinsäure | 19,56 | 0,8002 | 348,1 | 0,072 | 2,948 | 8,582 |
| Citraconsäure | 18,50 | 0,8003 | 397,0 | 0,050 | 3,051 | 6,505 |
| Itaconsäure | 22,83 | 0,8592 | 624,5 | 0,036 | 4,801 | 4,683 |

Im Falle der kontinuierlichen Coadsorptionen der Dicarbonsäuren Äpfel-, Citracon- und Itaconsäure in Anwesenheit von Glucose ist in allen drei Fällen ein vorzeitiger Durchbruch von Glucose zu beobachten, welcher durch die aus den Isothermen erhaltene, thermodynamisch bedingte, geringe Glucoseaufnahme zu erklären ist. Im Verlauf der Durchbruchskurve von Glucose ist weiterhin zu sehen, dass die Konzentration in allen Fällen kurz nach dem Glucosedurchbruch über die Konzentration des Feeds steigt, was durch Eintreten einer Teildesorption der Glucose durch Adsorption und Verdrängung der jeweiligen Säure zu erklären ist. Die Durchbrüche im Falle der Säuren sind in allen Fällen deutlich verzögert, verglichen mit Glucose. Die präferierte Adsorption der Säuren ist in diesem Falle wieder zu beobachten und ebenfalls konsistent mit den Ergebnissen der Batch-Adsorption.

**Tabelle 2.:** Daten der kontinuierlichen Coadsorption: Mit $c_0$ als Ausgangskonzentration, m als Masse des Adsorbens sowie $K_{th}$ als Kinetikkonstante und $q_{th}$ als Gesamtkapazität erhalten aus dem Thomas Modell.

| Substrat | $c_0$ [mg/mL] | $q_f$ [mL/min] | m [g] | $q_{th}$ [mg/g] | $K_{th}$ [mL/mg*min] | $q_{th}$ [mmol/g] | $K_{th}$ [ml/ mmol*min] |
|---|---|---|---|---|---|---|---|
| Äpfelsäure | 17,46 | 0,8 | 0,7918 | 198,0 | 0,045 | 1,477 | 5,98 |
| Glucose | 24,69 | 0,8 | 0,7918 | 163,1 | 0,457 | 0,90 | 82,26 |
| Citronsäure | 18,22 | 0,8 | 0,7952 | 287,2 | 0,041 | 2,208 | 5,33 |
| Glucose | 29,86 | 0,8 | 0,7952 | 195,2 | 0,042 | 1,084 | 7,56 |
| Itaconsäure | 16,003 | 0,8 | 0,7891 | 285,6 | 0,039 | 2,195 | 5,07 |
| Glucose | 25,50 | 0,8 | 0,7891 | 191,0 | 0,063 | 1,061 | 11,34 |

Die verwendete sphärische Aktivkohle (AC50) weist einen durchschnittlichen Partikeldurchmesser von 0.2 mm auf. Das Hochvernetzte Polymer wurde entsprechend der Vorschrift von Schute et al. synthetisiert. Folgende Materialeigenschaften wurden mittels $N_2$-Physisorption ermittelt:

**Tabelle 3:** Oberflächen und Volumina der Adsorbentien. Gemessen durch N2 Physisorption bei 77K

| Adsorbens | $S_{BET}$ [m²/g] | $S_{micro}$ [m²/g] | $V_{Ges}$ [cm³/g] |
|---|---|---|---|
| AC50 | 1889 | 1836 | 0,88 |
| HCP | 1842 | 513 | 1,73 |

**Tabelle 4:** Isothermen-Parameter der Kompetitivadsorptionen von Itacon-, Äpfel-, Bernstein- und Maleinsäure an AC50 und HCP.

| Substrat | AC50 | | | | HCP | | | |
|---|---|---|---|---|---|---|---|---|
| | $K_f$ | n | $q_{mon}$ | b | $K_f$ | n | $q_{mon}$ | b |
| Itaconsäure | 4,50 | 0,18 | 3,54 | 120,29 | 4,41 | 0,34 | 3,47 | 16,60 |
| Glucose | 0,06 | -0,53 | 0,20 | 86346 | -0,30 | 2,50 | 0,00 | 0,00 |
| Äpfelsäure | 3,59 | 0,27 | 2,75 | 27,43 | 4,82 | 1,13 | 4902 | 0,00 |

(fortgesetzt)

| Substrat | AC50 | | | | HCP | | | |
|---|---|---|---|---|---|---|---|---|
| | $K_f$ | n | $q_{mon}$ | b | $K_f$ | n | $q_{mon}$ | b |
| Glucose | 0,08 | -0,41 | 0,21 | 71282 | 0,02 | 0,42 | 0,05 | -94,19 |
| Bernsteinsäure | 4,43 | 0,22 | 3,33 | 72,44 | 4,13 | 0,51 | 3,25 | 7,12 |
| Glucose | 0,01 | -1,05 | 0,09 | -9*10⁶ | 0,01 | -0,11 | 0,01 | 144,3 |
| Maleinsäure | 4,76 | 0,25 | 3,36 | 61,24 | 6,52 | 0,91 | 12,01 | 0,73 |
| Glucose | 0,01 | -0,89 | 0,06 | -99,55 | 0,02 | -0,30 | 0,04 | 94245 |

Ferner wurden die Dipolmomente der verwendeten Dicarbonsäuren bestimmt. Die Dipolmomente der Dicarbonsäure und der Glucose wurden mit Gamess US berechnet. Als Methode wurde eine DFT (B3LYP) mit einem 6-31G Basissatz verwendet. Dazu wurden die Moleküle zuvor mit einem MM2 Kraftfeld geometrisch optimiert. Alle Dipolmomente wurden zweifach bestimmt.

Ferner wurden Batch Adsorptionsexperimente durchgeführt. Die Bath Adsorptionsexperimente wurden in einem Wasserschüttelbad (GFL) mit reziproker Bewegung und verstellbarer Temperatur durchgeführt. Es wurden Adsorptionsisothermen der verschiedener Adsorbentien (PBSAC (AC50) und HCP) mit den jeweiligen Dicarbonsäuren, sowie Mischungen von Dicarbonsäuren und Glucose aufgenommen. Die Adsorptionsexperimente wurden bei 20 °C und mit unterschiedlichen Anfangskonzentrationen (maximal 50 mg/g) der Dicarbonsäuren (Itaconsäure, Maleinsäure, Bernsteinsäure, Methylbernsteinsäure, Äpfelsäure, Weinsäure, Mesaconsäure, Citraconsäure), sowie der Glucose als reine Komponente oder als Mischung durchgeführt. In einem klassischen Adsorptionsexperiment wurden 40 mg Adsorbens zu 2g Lösung entsprechender Konzentration gegeben, wobei die Mischung bei isothermen Bedingungen gehalten wurde. Die Adsorptionsexperimente wurden über eine Länge von einer Stunde durchgeführt, sodass ein Gleichgewichtszustand gewährleistet war. Die Beladung des Adsorbens im Gleichgewicht ($q_{eq}$) wurde dann wie folgt berechnet:

$$q_{eq} = \frac{n_{adsorbiert}}{m_{adsorbens}} = \frac{(c_i - c_e) \cdot m_{ges}}{M \cdot m_{adsorbens}}$$

Dabei ist n die Stoffmenge an adsorbiertem Adsorptiv, $m_{adsorbens}$ die Masse des Adsorbens, $c_i$ die Konzentration an Adsorptiv vor der Adsorption, $c_e$ die Konzentration an Adsorptiv nach der Adsorption, $m_{ges}$ die Masse der Lösung und M die molare Masse des Adsorptivs.

Die kompetitive Adsorption der Dicarbonsäuren aus einem Dicarbonsäure/ Glucose/ Wasser Gemisch wurde mit äquimolaren Lösungen (Dicarbonsäure : Glucose = 1:1) durchgeführt. Die Parameter der Isothermen-Modelle wurden mit SciDaVis durch eine nicht lineare Regression ermittelt.

Alle erhaltenen Isothermen wurden mithilfe etablierter Isothermenmodelle charakterisiert. Als empirisches Modell wurde das Freundlich Modell verwendet.

$$q(T) = K_f(T) \cdot c_{eq}^n$$

Dabei ist $q(T)$ die temperaturabhängige Gleichgewichtsbeladung des Adsorbens [$g_{adsorptiv}$/$g_{adsorbens}$], $K_f(T)$ der Freundlich-Koeffizient [mmol $g_{ads}$($g_{solv}$ mmol)$^{1/n}$], $c_{eq}$ die Gleichgewichtskonzentration des Adsorptivs [mmol/g] und $n$ ein empirischer Exponent.

Zudem wurde das auf thermodynamische Grundlagen basierende Langmuir Modell verwendet, welches einen Informationsgehalt über die Monolagenkapazität eines Adsorbens sowie den Adsorptionskoeffizienten liefert.

$$q(T) = q_{mon}(T) \cdot \frac{b(T) \cdot c_{eq}}{1 + b(T) \cdot c_{eq}}$$

Dabei ist $q_{mon}(T)$ die temperaturabhängige monomolekulare Beladung des Adsorbens [mmol/g], und b(T) der Langmuir-Sorptionskoeffizient [$g_{solv}$/mmol]. Der Langmuir-Sorptionskoeffizient lässt sich als der Quotient aus Adsorptionsgeschwindigkeit und Desorptionsgeschwindigkeit (b(T)=$k_{ads}$/$k_{des}$) verstehen und gibt damit die Bindungsaffinität des Adsorptivs auf der Oberfläche des Adsorbens an. Die monomolekulare Beladung gibt die maximale Kapazität des Adsorbens für das jeweilige Adsorptiv an.

Für die Messung von Durchbruchskurven wurde als Adsorbens AC50 Kugelkohlen in einem Festbett verwendet. Eine Stammlösung mit 2 w% Adsorptiv wurde bei einem konstanten Volumenstrom von 0.8 mL/min mit einer HPLC-Pumpe (LC-20AT, Shimadzu) durch die Schüttung gefördert. Es wurde eine Totzeit von 75 Sekunden ermittelt, sodass dann die erste Probennahme erfolgte. Am Ausgang des Festbetts wurden dann Proben entnommen, deren Konzentrationen mittels HPLC bestimmt wurden. Jede Probe gibt dabei den Mittelwert der Konzentration über den Zeitraum, in dem sie aufgefangen wurde. Es wurden Durchbruchkurven sowohl nur Reinstofflösungen, als auch mit Dicarbonsäure-Glucose-Gemischen gemessen.

Ein bewährtes Modell zur Bestimmung der Kapazität und Kinetik des Systems, sowie zur Modellierung von Durchbruchkurven, ist das Thomas Modell, welches durch folgende Gleichung gegeben ist:

$$\frac{c_e}{c_0} = \frac{1}{1 + \exp(-K_{th} \cdot c_0 \cdot t + \frac{K_{th} \cdot q_{th} \cdot m}{q_f})}$$

Dabei ist $c_e$ die Konzentration am Ausgang der Schüttung [mg/mL], $c_0$ die Startkonzentration [mg/mL], $K_{th}$ die Kinetik Konstante des Thomas Modells auf Grundlage einer reversiblen Reaktion zweiter Ordnung [mL/mg*min], $t$ die Zeit der Probennahme [min], m die Masse an Adsorbens [g], $q_f$ die Flussrate [mL/min] und $q_{th}$ die Beladung des Adsorbens mit Adsorptiv [mg/g].

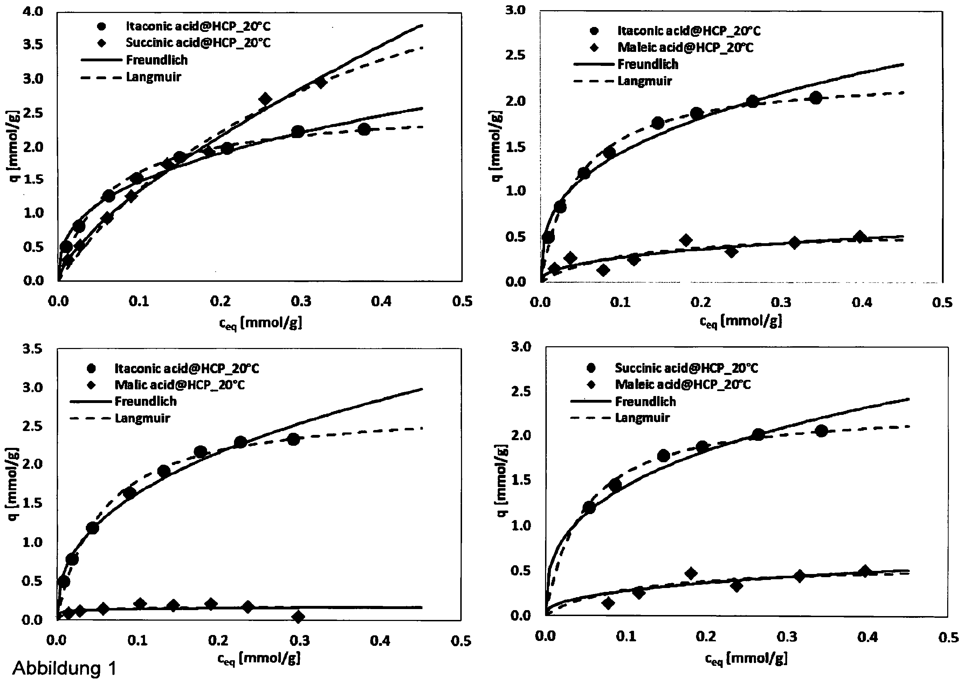

Die folgenden Abbildungen zeigen die durchgeführten Messungen zur Durchführung des beschriebenen Verfahrens: Abbildung 1 zeigt die Konkurrenzadsorption jeweils zwei verschiedener Dicarbonsäurenan dem porösen Polymer HCP, insb. Polymethylenbiphenyl, als hydrophobem Adsorbens. Die Verbindungen besitzen eine unterschiedliche Polarität wodurch jeweils die Dicarbonsäure mit der geringeren Polarität in signifikant höheren Mengen adsorbiert werden, als die polarere Glucose. Aufgetragen ist die adsorbierte Menge in Abhängigkeit der vorliegenden Gleichgewichtskonzentrationen der beiden Komponenten in der wässrigen Lösung. Die Abbildung oben links zeigt die Adsorption von Itakon- und Bersteinsäure, wobei bei Konzentrationen größer als 0.2 mmol/g deutlich mehr Bernsteinsäure (Succinic Acid) adsorbiert wird. Die Abbildung oben rechts zeigt die bevorzugte Adsorption über den gesamten Konzentrationsbereich von Itakonsäure gegenüber Maleinsäure, unten links von Itakonsäure gegenüber Äpfelsäure (Malic Acid) und unten rechts Bernsteinsäure gegenüber Maleinsäure. Alle experimentell gemessenen Isothermen wurden mittels Freundlich- und Langmuir-Isothermenmodell mathematisch beschrieben.

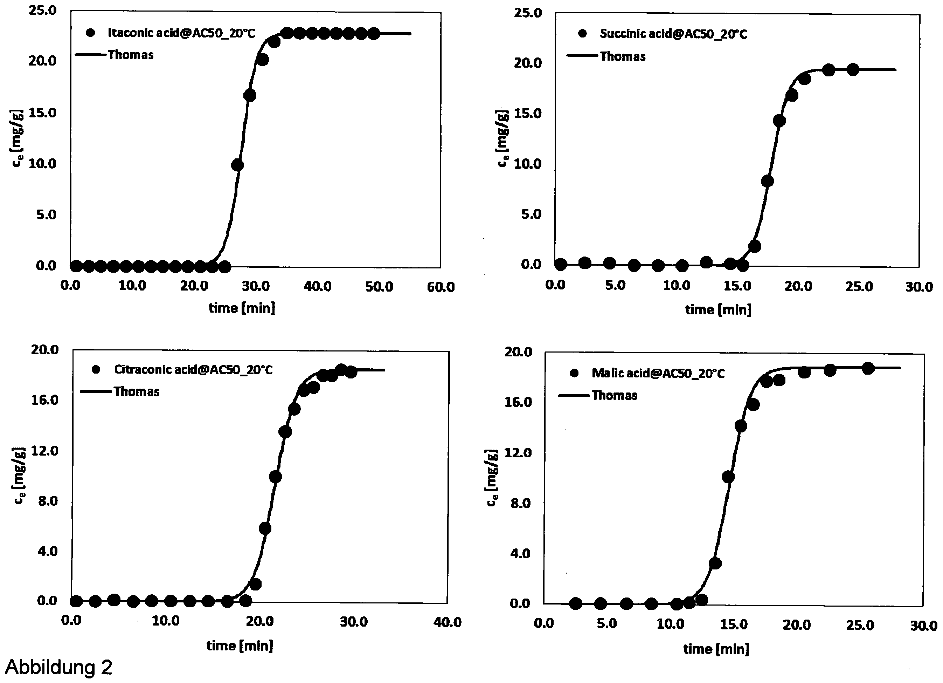

Abbildung 2 zeigt die Durchbruchskurven einzelner Dicarbonsäuren aus 2Gew-%-igen wässrigen Lösungen bei 20°C nach Adsorption in einer mit der Aktivkohle AC50 gepackten Adsorberkolonne. Die Durchbruchskurven wurden mathematisch mit dem Thomas-Modell beschrieben. Aufgetragen ist die Konzentration der Säure am Ausgang der Adsorberkolonne nach der Adsorption gegen die Zeit bei konstantem Volumenstrom durch die Kolonne. Die Abbildung oben links zeigt Itakonsäure, oben rechts Bernsteinsäure, unten links Citraconsäure und unten rechts Äpfelsäure.

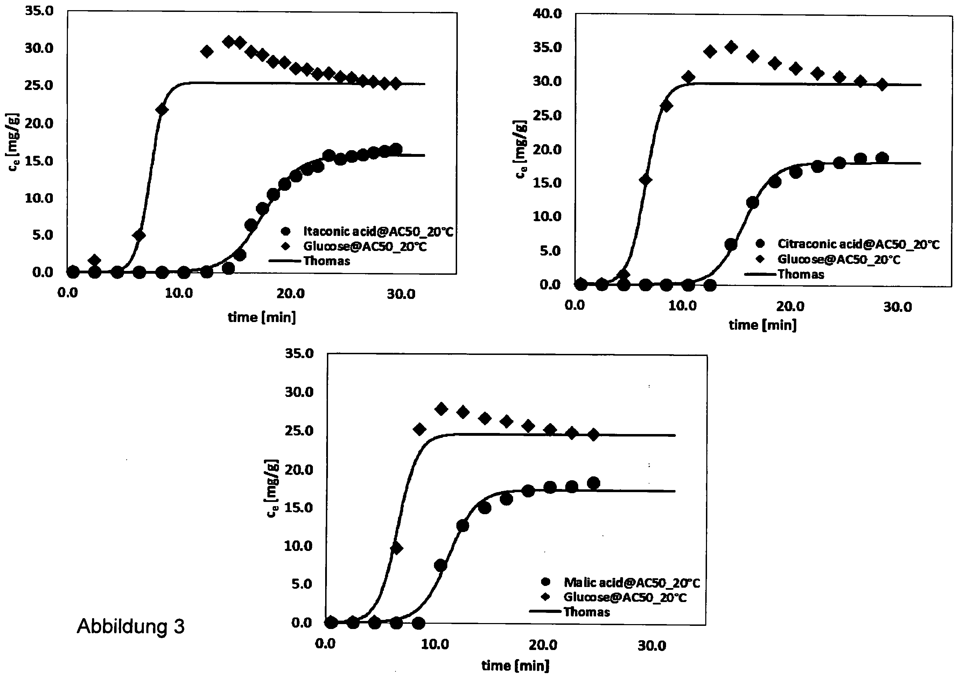

Abbildung 3 zeigt die kontinuierliche Co-Adsorption-Durchbruchskurven von 2 Gew.-%-igen Lösungen aus den Gemischen Itaconsäure/Glucose (oben links), Citraconsäure/Glucose (oben rechts), Apfelsäure/Glucose (unten) an der Aktivkohle AC50. In allen Beispielen erfolgt der Durchbruch der weniger adsorbierenden Komponente Glucose deutlich vor dem Durchbruch der jeweiligen Dicarbonsäuren, was zeigt, dass die Kapazität für die früher durchbrechende Komponente deutlich geringer ist. Im Falle der Coadsorption kann zudem eine Abnahme der Gesamtkapazität beobachtet werden. Alle $q_{th}$-Werte sind deutlich geringer als in der Einkomponentenadsorption, wobei eine Selektivität weiterhin besteht (Tabelle 2). Des Weiteren fällt auf, dass durch die Anwesenheit der Glucose der Stofftransport der Säuren verlangsamt wird, was aus den Transportkoeffizienten des Thomas-Modells ($K_{th}$) hervorgeht.

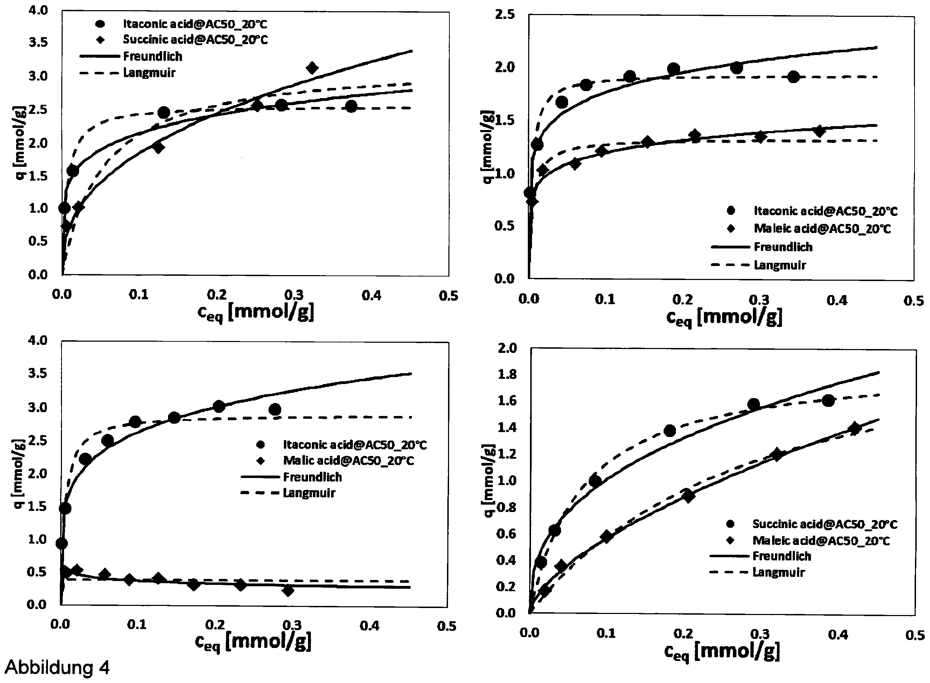

Abbildung 4 zeigt die Konkurrenzadsorption jeweils zwei verschiedener Dicarbonsäurenan der Aktivkohle AC50, als hydrophobem Adsorbens. Die Verbindungen besitzen eine unterschiedliche Polarität wodurch jeweils die Dicarbonsäure mit der geringeren Polarität in signifikant höheren Mengen adsorbiert werden, als die polarere Glucose. Aufgetragen ist die adsorbierte Menge in Abhängigkeit der vorliegenden Gleichgewichtskonzentrationen der beiden Komponenten in der wässrigen Lösung. Die Abbildung oben links zeigt die Adsorption von Itakon- und Bersteinsäure, wobei bei Konzentrationen größer als 0.2 mmol/g deutlich mehr Bernsteinsäure (Succinic Acid) adsorbiert wird. Dieser Effekt fällt aufgrund der geringeren Hydrophobie von AC50 im Vgl. zu HCP (Abb. 1) geringer aus. Die Abbildung oben rechts zeigt die bevorzugte Adsorption über den gesamten Konzentrationsbereich von Itakonsäure gegenüber Maleinsäure, unten links von Itakonsäure gegenüber Äpfelsäure (Malic Acid) und unten rechts Bernsteinsäure gegenüber Maleinsäure. Alle experimentell gemessenen Isothermen wurden mittels Freundlich- und Langmuir-Isothermenmodell mathematisch beschrieben. Steigt die Polaritätsdifferenz zwischen den Säuren, so ist zu erkennen, dass die Säure mit geringerer Polarität bevorzugt adsorbiert wird. Wobei die Ausprägung der Selektivität mit steigendem Polaritätsunterschied zunimmt. Im System Itaconsäure/Bernsteinsäure adsorbieren beide Säuren nahezu gleichermaßen stark, während im Fall Itaconsäure/Äpfelsäure nahezu ausschließlich Itaconsäure adsorbiert wird. Im Falle der Konkurrenzadsorption von Bernsteinsäure und Maleinsäure deren Polaritätsdifferenz sehr gering ist, kann ebenfalls eine Selektivität hin zur unpolaren Substanz Bernsteinsäure beobachtet werden. Eine beachtliche Veränderung der Adsorptionskapazitätkann für den Fall der AC50 nicht beobachtet werden.

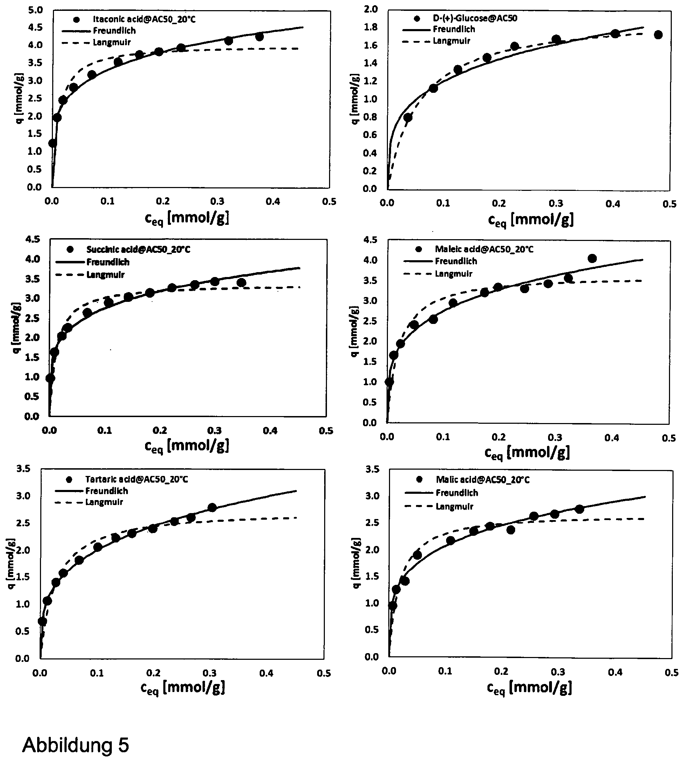

Abbildung 5a - 5f zeigt Einkomponentenisothermen aus wässrigen Lösungen bei 20°C an AC50. Die Isothermen wurden jeweils mit den Freundlich- und Langmuir-Isothemenmodellen modelliert. Die Abbildung 5a zeigt die Adsorptioinsisotherme von Itakonsäure, Abbildung 5b Glucose, Abbildung 5c Bernsteinsäure, Abbildung 5d Maleinsäure, Abbildung 5e Weinsäure (Tartaric Acid) und Abbildung 5f Äpfelsäure (Malic Acid). Der Verlauf der Isothermen ist für alle Komponenten vergleichbar. Mit zunehmender Gleichgewichtskoncentration steigt die adsorbierte Menge bezogen auf die Adsorbensmasse.

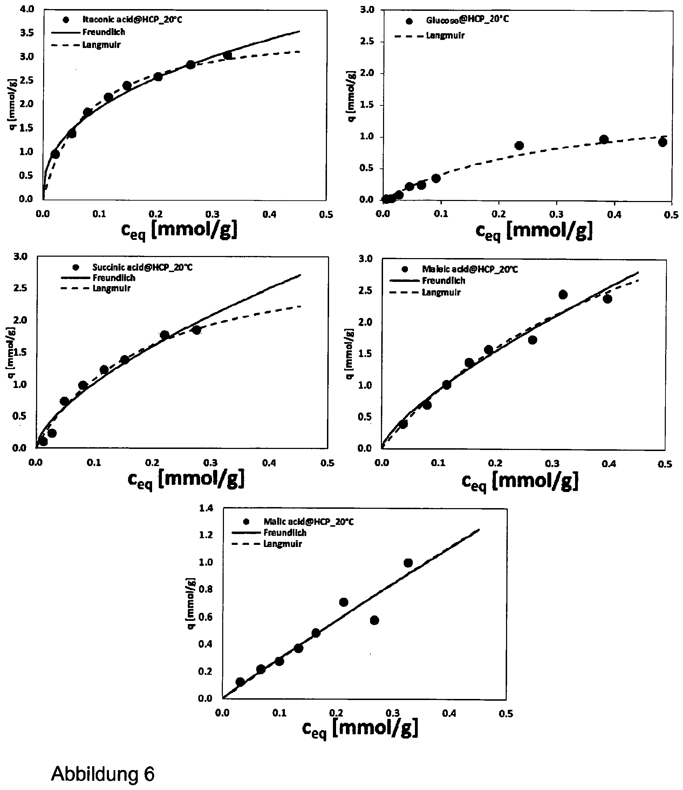

Abbildung 6a - 6e zeigt Einkomponentenisothermen aus wässrigen Lösungen bei 20°C an dem porösen Polymer HCP (Polymethylenbiphenyl). Modellierungen wurden jeweils mit den Freundlich und Langmuir Modellen durchgeführt. Die Abbildung 6a zeigt die Adsorptioinsisotherme von Itakonsäure, Abbildung 6b Glucose, Abbildung 6c Bernsteinsäure, Abbildung 6d Maleinsäure, Abbildung 6e Äpfelsäure (Malic Acid). Der Verlauf der Isothermen ist für alle Komponenten vergleichbar. Mit zunehmender Gleichgewichtsconcentration steigt die adsorbierte Menge bezogen auf die Adsorbensmasse.

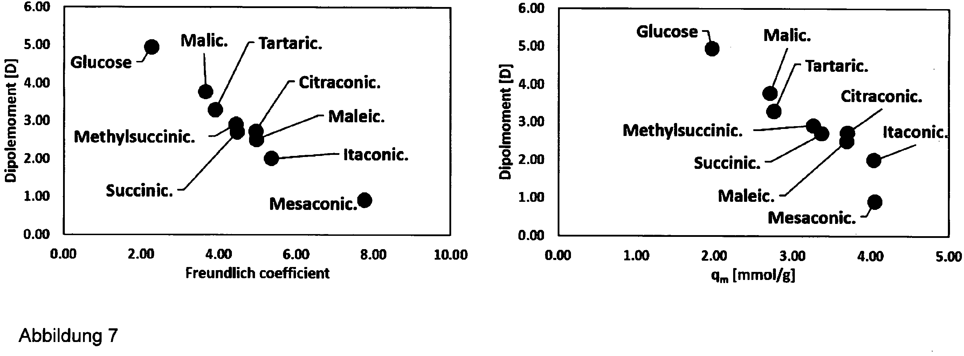

Abbildung 7 zeigt die direkte Korrelation der aus der Modellierung der Adsorption an der Aktivkohle AC50 bestimmten Konstanten (die Maximalbeladung einer theoretischen Monolage aus dem Langmuir-Modell ($q_m$, rechte Abbildung) sowie die Adsorptionskonstante aus dem Freundlich-Modell ($K_F$, linke Abbildung) sowie der berechneten Dipolmomente der unterschiedlichen DIcarbonsäuren. Die Korrelation zeigt, dass unpolare Komponenten wie etwa Itacon-, Mesacon- oder etwa Maleinsäure bevorzugt und mit hoher Kapazität adsorbieren, während polarere Säuren aber auch Glucose mit deutlich geringerer Kapazität adsorbiert werden.Gleiches, nur wesentlich ausgeprägter ist für HCP zu beobachten. Es wird deutlich, dass unpolare Säuren mit hoher Beladung und somit bevorzugter adsorbieren als polare Komponenten. Auffällig ist hingegen, dass die Beladungsspannweite für HCP deutlich größer ist im Vergleich zur AC50. Dies führt unmittelbar zur Schlussfolgerung, dass HCP sensitiver auf Polaritätsunterschiede reagiert als etwa AC50, was ein erstes Indiz zu einer möglichen Polaritätsselektion liefert.

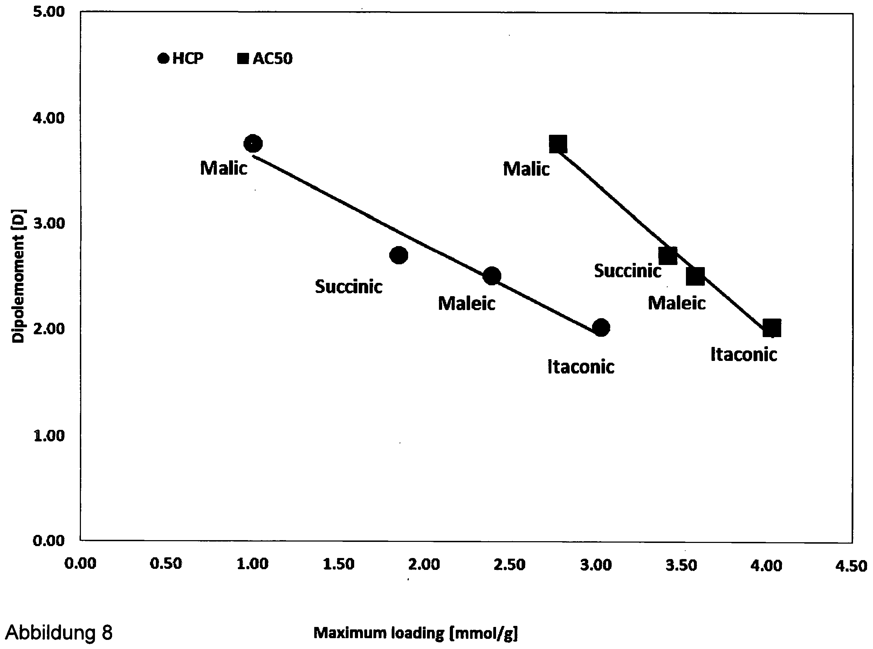

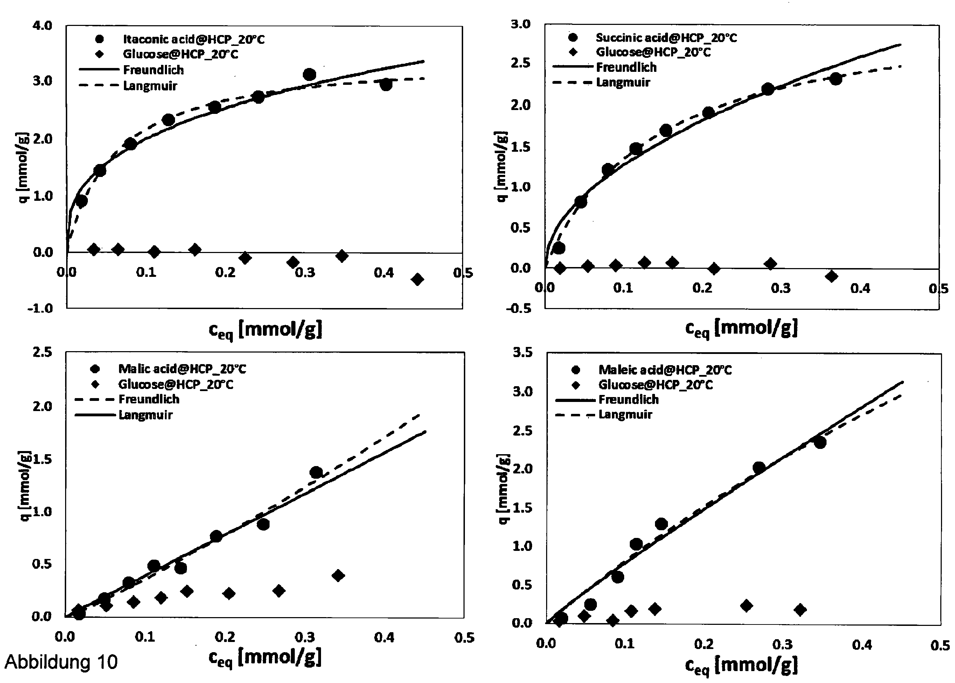

Abbildung 8 zeigt die Auftragung der Maximalbeladung der Adsorption verschiedener Dicarbonsäuren gegen das Dipolmoment für HCP und AC50. Dargestellt sind die Datenpunkte für die ausgewählten Säuren Itacon-, Malein-, Bernstein- und Apfelsäure. So zeigt Abbildung 10 die Gesamtaufnahmekapazität von HCP, welche im Vergleich zur AC50 zwar geringfügig niedriger ist; so beträgt die Maximalaufnahme von Itaconsäure etwa 3.0 mmol g$^{-1}$, die Selektivität zur jeweiligen Säure ist jedoch deutlich größer. In den vier Kompetitivadsorptionsexperimenten geht die Aufnahme von Glucose durch HCP nahezu gegen Null. Steigt die Polarität der Säure, so nimmt auch die Gesamtaufnahme dieser ab. Beim Vergleich der Adsorption von Bernsteinsäure an HCP mit Äpfelsäure an HCP, wird im Falle von Bernsteinsäure eine Kapazität von etwa 2.5 mmol g$^{-1}$ erreicht, im Falle der Äpfelsäure eine Kapazität von etwa 1.5 mmol g$^{-1}$. In Kontrast zur Aktivkohle AC50 wirken sich somit Polaritätsunterschiede deutlich stärker auf das Adsorptionsverhalten von HCP aus, welches bislang noch bei keinem anderen Material derart beobachtet wurde.

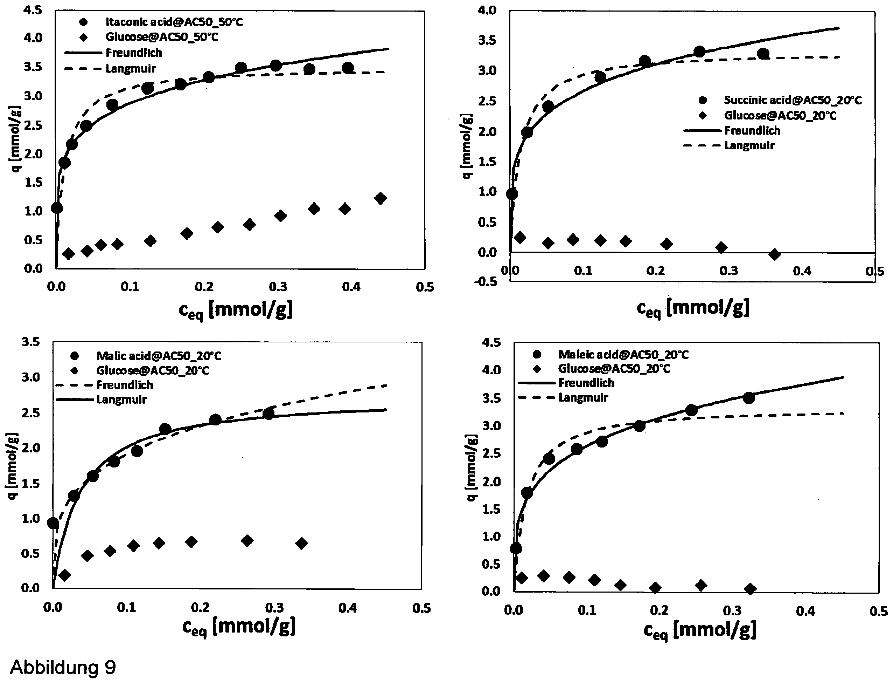

Abbildung 9 zeigt Konkurrenzadsorption verschiedener Dicarbonsäure/Glucose Mischungen unterschiedlicher Polarität an kommerzieller Aktivkohle AC50. (oben links: Itaconsäure; oben rechts:Bernsteinsäure; unten rechts:Maleinsäure; unten links:Apfelsäure). Die Glucoseadsorption als Reinstoffisotherme dagegen (siehe auch Abbildung 5) weisen eine Aufnahme bis zu 2.0 mmol g$^{-1}$ auf und ist respektive signifikant höher als im Mehrkomponentensystem. Somit sind die hydrophoben Adsorbentien wie AC50 eindeutig selektiver für die Adsorption unpolarerer Komponenten wie der untersuchten Dicarbonsäuren im Vergleich zur Glucose.

Abbildung 10 zeigt die Konkurrenzadsorption jeweils verschiedener Dicarbonsäuren und ihrem Substrat Glucose an dem porösen Polymer HCP (Polymethylenbiphenyl) als hydrophobes Adsorbens. Die Verbindungen besitzen eine unterschiedliche Polarität wodurch jeweils die Dicarbonsäure in signifikant höheren Mengen adsorbiert werden, als die polarere Glucose, die z.T. über einen breiten Konzentrationsbereich gar nicht adsorbiert wird. Aufgetragen ist die adsorbierte Menge in Abhängigkeit der vorliegenden Gleichgewichtskonzentrationen der beiden Komponenten in der wässrigen Lösung. Die Abbildung links oben zeigt Die Co-Adsorption Itakonsäure-Glucose, rechts oben Bernsteinsäure-Glucose, links unten Äpfelsäure-Glucose und rechts unten Maleinsäure-Glucose.

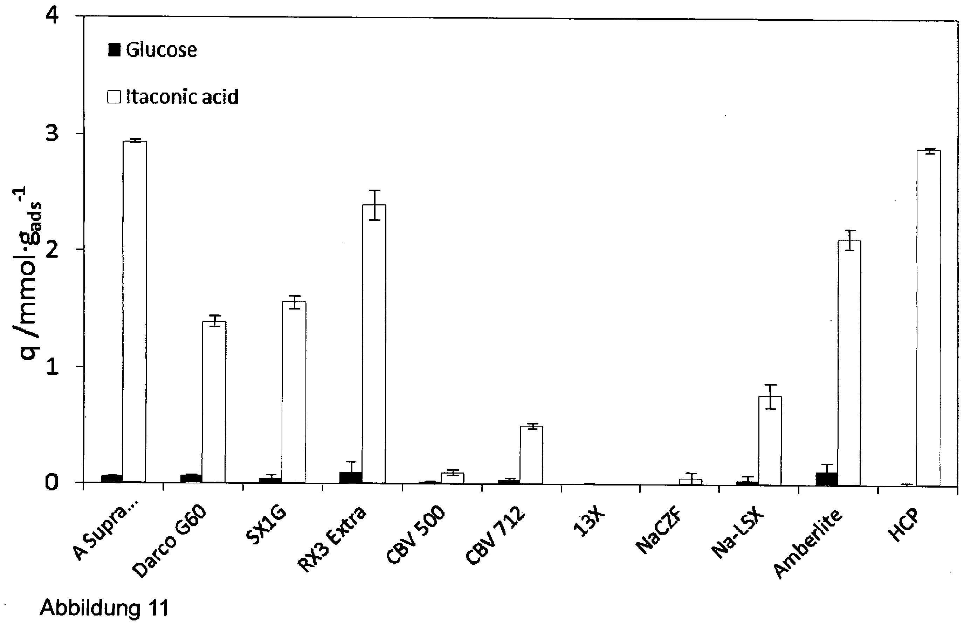

In Abbildung 11 ist zu erkennen, dass die besten Ergebnisse für eine Adsorption von Itaconsäure durch die Aktivkohlen A Supra EUR, RX3 Extra und HCP als Adsorbentien erhalten wurden. Der Anteil hoher und selektiver Adsorption von Aktivkohle kann mit der hohe spezifische Oberfläche und das Porenvolumen mit niedriger Polarität in Zusammenhang gebracht werden. Demgegenüber zeigen Zeolithe wie CBV 500, CBV 712, 13 X, NaCZF und Na-LSX schlechteres Adsorptionsverhalten in Bezug auf die Adsorption von Itaconsäure in Gegenwart von Glucose. Die geringe Affinität der Zeolithe für Itaconsäure kann mit den geringeren spezifischen Oberflächen und Gesamtporenvolumen sowie der hohen Polarität der Oberfläche dieser Adsorbentien erklärt werden.

Demgegenüber zeigt Amberlite als ein schwaches basisches Anionenaustauscharz bestehend aus Styrol Divinylbenzol-Copolymer mit basischen Amingruppen, trotz niedrigster spezifischer Oberfläche und niedrigstem Gesamtporenvolumen von allen getesteten Materialien nur eine geringfügig geringere Adsorption von Itaconsäure im Vergleich zu den ausgewerteten Aktivkohlen, was durch direkte Säure-Base-Wechselwirkungen zwischen Adsorbens und den Adsorbentien erklärt werden kann. Die Adsorptionskapazität für Itaconsäure von Amberlite ist mit den hochvernetzten Polymeren HCP als Adsorbentien vergleichbar. Es ist jedoch zu erkennen, dass die höchste Selektivität für Itaconsäureadsorption gegenüber Glucose mit HCP-Adsorbentien erzielbar ist, was auf die hohe Hydrophobie des HCP aufgrund seiner unpolaren organische Netzwerkstruktur zurückzuführen sein könnte.

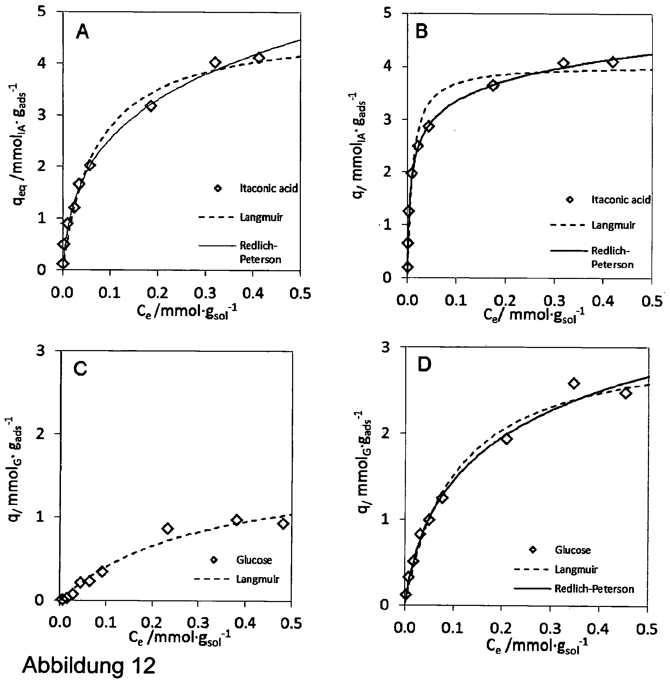

Abbildung 12a - 12d zeigt eine Übersicht von Adsorptionsisothermen von Itaconsäure und Glucose für HCP und A Supra EUR gemessen, wobei die Isothermen-Modelle von Langmuir und Redlich-Peterson zugrunde gelegt wurden, um die gemessenen Daten durch entsprechende Isothermen zu modellieren.

In Abbildung 12a ist die Adsorptionsisotherme von Itaconsäure für die Adsorption von HCP und in Figur 12b für die Adsorption an A Supra EUR dargestellt. Die Adsorptionsisothermen von Glucose sind in Figur 12c für die Adsorption an HCP und in Figur 12d für die Adsorption an A Supra EUR dargestellt. Ein Vergleich der Kurven zeigt, dass für HCP und A Supra EUR bei der Itaconsäure Adsorption eine nahezu gleiche Adsorptionskapazität erhalten wird. Bei der Adsorption von Glucose unterscheiden sich die Adsorptionskapazitäten jedoch erheblich. So werden bei der Adsorption von Glucose an HCP gegenüber der Adsorption von Glucose an A Supra EUR geringere Adsorptionskapazitäten gefunden. Diese Abweichungen können auf eine hohe Konzentration von polaren Gruppen der Glucose-Moleküle zurückzuführen sein. A Supra EUR ist in der Lage, eine größere Menge (2,5 mmol/g) Glucose als HCP (1 mmol/g) an der Oberfläche zu adsorbieren, was auf die Anwesenheit von einigen polaren funktionellen Gruppen auf der Oberfläche der Aktivkohle zurückzuführen ist.

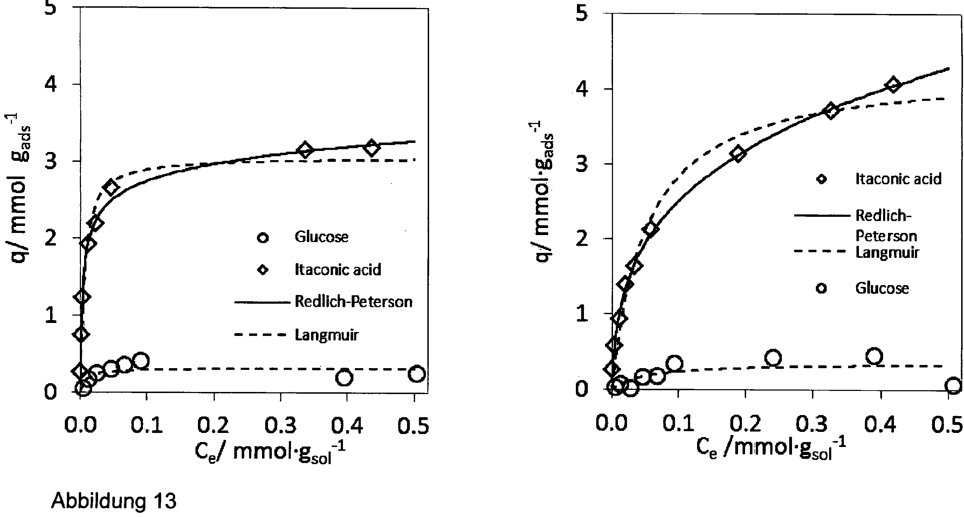

In Abbildung 13 sind Mehrkomponenten-Adsorptions-Isothermen von Itaconsäure und Glukose dargestellt. Um das Adsorptionsverhalten in realen Fermentationslösungen angenähert darzustellen, wurde eine konkurrierende Adsorption von Itaconsäure und Glucose ausgewählt und eine Mehrkomponenten-Lösung untersucht. Es zeigt sich eine abnehmende Adsorptionstendenz mit zunehmender Konzentration zwischen HCP und A Supra EUR aufgrund der Adsorptionskonkurrenz zwischen beiden Adsorbaten. Im Allgemeinen hängt die Adsorption aus einer flüssigen Phase von den Wechselwirkungen zwischen den Adsorbentien und Adsorbat, Adsorbat-Adsorbat, Lösungsmittel-Adsorbens und Lösungsmittel-Adsorbat ab, was zu einem komplexen Adsorptionssystem führt. Die erhaltenen Langmuir Isothermen sind daher gegenüber den Werten von Figur 12a und 12b bei der Adsorption von Itaconsäure und Glucose an HCP bzw. A Supra EUR zu geringeren Werten hin verschoben. Die Adsorptionskapazitäten der Itaconsäure verringert sich in der Mehrkomponenten-Lösung im Vergleich zu der Einzelkomponenten-Lösung, was auf die konkurrierende Adsorption der Glucose zurückzuführen ist. Die Hydrophobie der aromatischen Struktur des HCP führt zu einer selektiven Adsorption, was durch Erreichen einer Sättigungskapazität schon bei geringen Glucosekonzentrationen belegt werden kann. Die Aktivkohle A Supra EUR besteht demgegenüber aus einem amorphen Kohlenstoffmaterial mit einigen hydrophilen Gruppen (sauerstoffhaltigen Gruppen), die auf eine höhere Affinität zu Glucose hinweisen. Die Ausbildung eines von der Glucosekonzentration unabhängigen Gleichgewichtszustands ist dadurch bei HCP begünstigt.

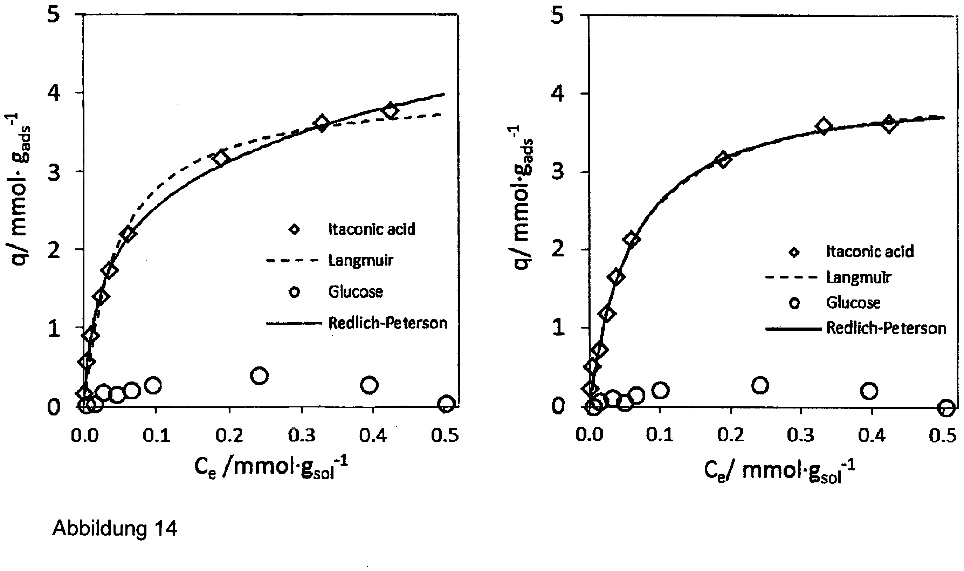

Abbildung 14 zeigt den Einfluss der Temperatur auf die Adsorptionskapzität von Itaconsäure in einem Mehrkomponenten-System im Fall von HCP als Adsorbens. Es wurden Adsorptions-Isothermen bei verschiedenen Temperaturen (50°C und 80°C) erstellt, um den Einfluss der Temperatur und der Itaconsäureadsorption und der Stärke der Wechselwirkung zwischen Adsorbens und Adsorbat auszuwerten. Im Allgemeinen kann festgestellt werden, dass der Einfluss der Temperatur auf die Adsorption nur gering ist und mit vorherigen Ergebnissen in Bezug auf das Adsorptionsverhalten übereinstimmt.

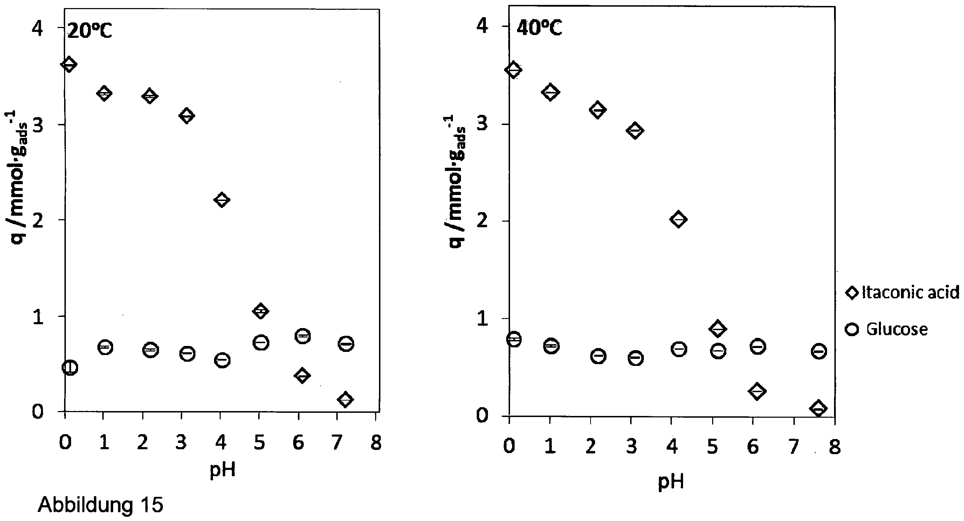

In Abbildung 15 ist der Einfluss des pH-Wertes auf die Adsorption von Itaconsäure und Glucose in einem Mehrkomponentensystem an HCP dargestellt. Itaconsäure ist gekennzeichnet als eine organische Säure mit zwei Säurefunktionen. Aufgrund des Dissoziationsverhaltens der Itaconsäure ist der Einfluss des pH-Wertes auf die Adsorption in Gegenwart von Glucose stark ausgeprägt. Der Einfluss des pH-Wertes bei der Adsorption ist in Fig. 15 bei 20°C und 40 ° C dargestellt. Bei Erhöhung eines anfänglich niedrigen pH-Werts der Lösung von pH 0 bis 8, ist eine deutliche Abnahme der Adsorptionskapazität zu verzeichnen, wobei der Einfluss der Temperatur auf das Adsorptionsverhalten nur gering ist. Andererseits ist die GlucoseAdsorption bei den ausgewerteten Temperaturen konstant, und verändert sich auch nicht mit der Zunahme des pH-Wertes von 0 bis 8. Eine weitere Abnahme der pH-Werte, von 2 auf 0 führt ist keiner entscheidenden Erhöhung der Adsorptionskapazität mehr.

Die pH Auswertungsergebnisse zeigen, dass protonierte und nicht in ionischer Form vorliegende Itaconsäure bevorzugt auf der HCP Oberfläche adsorbiert wird. Steigerung des pH-Werts der Lösung hat zur Folge, dass die Konzentration von deprotonierter Itaconsäure erhöht wird und die Adsorptionsmenge aufgrund der schwachen Wechselwirkung zwischen der unpolaren HCP-Oberfläche und den polaren Itaconat-Ionen abnimmt.

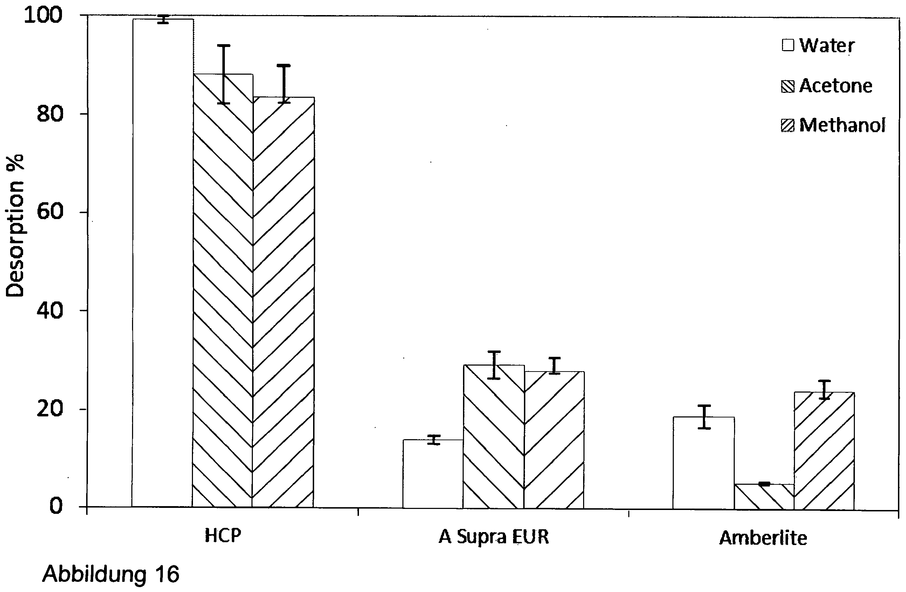

Abbildung 16 zeigt schließlich das Desorptions-Verhalten von Itaconsäure an HCP. Zur Durchführung der Versuche wurden drei verschiedene Lösungsmittel für die Desorption von Itaconsäure und Glucose aus HCP, A Supra EUR und Amberlite verwendet. Das erste getestete Desorptions-Lösungsmittel war Wasser. Zusätzlich wurde Methanol getestet, da es bekannt ist, dass es die höchste Löslichkeit von Itaconsäure in kommerziellen Lösungsmitteln zeigt. Auch Aceton wurde wegen seiner guten Lösungseigenschaften von Itaconsäure eingesetzt.

Es zeigt sich, dass die Desorption am effizientesten an HCP war, da fast die gesamte Menge an adsorbierter Itaconsäure unter Verwendung von Wasser als Lösungsmittel desorbiert wurde. Grund dafür ist, die hohe Affinität zwischen den beiden Carboxylgruppen der Itaconsäure und Wasser. Diese ist stark genug ist, um die Desorption der Itaconsäure zu begünstigen. Methanol kann ebenfalls durch seine hohe Polarität und die guten Lösungseigenschaften der Itaconsäure ebenso wie Aceton, große Mengen von Itaconsäure von HCP desorbieren. Im Gegensatz dazu zeigen A Supra EUR und Amberlite weniger als 30% Freisetzung von Itaconsäure bei der Verwendung von Wasser, Methanol und Aceton, was auf die starke Affinität zwischen Adsorbens und Adsorbat im Vergleich zu HCP zurückzuführen sein könnte. Dies führt zu der Annahme, dass eine Chemisorption aufgrund der funktionellen sauerstoffhaltigen Gruppen an der Oberfläche und durch die Ausbildung von Säure-Base-Wechselwirkungen eine Desorption wie bei Aktivkohle und Amberlite erschwert.

## Patentansprüche

1.  Verfahren zur Abtrennung mehrerer Dicarbonsäuren oder Fraktionen mehrerer Dicarbonsäuren aus biologischen und/oder chemischen Reaktionslösungen durch Adsorption bei einem pH-Wert 1 bis 7 in einem Temperaturbereich von 20°-80°C an zumindest teilweise unpolare, hydrophobe Adsorbentien, von denen nachfolgend ein Adsorbat desorbiert wird, **dadurch gekennzeichnet,**
    **dass** die Adsorbentien High Crosslinked Polymers (HCP) umfassen, welche ausgewählt sind aus Polymethylenbiphenyl oder Polymethylenbenzol wobei eine Trennung von mehreren koexistierenden Dicarbonsäuren durch selektive Adsorption erfolgt, wobei die Dicarbonsäuren ausgewählt sind aus Malein-, Itacon-, Mesacon-, Citracon-, Methylbernstein-, Wein- und Äpfelsäure, und die adsorbierten Dicarbonsäuren von den Adsorbentien bei Eluierung mit Wasser, Methanol, Ethanol oder Aceton erhalten werden.

2.  Verfahren nach Anspruch 1,
    **dadurch gekennzeichnet,**
    **dass** das Porenvolumen der Adsorbentien eine Größe von 0,01cm$^3$/g bis 4,0 cm$^3$/g und die Adsorbentien eine Oberfläche im Bereich von 100 m$^2$/g bis 4000 m$^2$/g umfassen

3.  Verfahren nach einem der voranstehenden Ansprüche,
    **dadurch gekennzeichnet,**
    **dass** die Adsorption in einem pH Bereich zwischen 2 und 5 erfolgt.

4.  Verfahren nach einem der voranstehenden Ansprüche,
    **dadurch gekennzeichnet,**
    **dass** die Desorption bei einem pH-Wert im Bereich größer oder gleich 7 erfolgt.

5.  Verfahren nach einem der voranstehenden Ansprüche,
    **dadurch gekennzeichnet,**
    **dass** in äquimolaren Lösungen von Dicarbonsäuren und Glucose weniger als 1mmol Glucose pro g der Adsorbentien adsorbiert wird.

## Claims

1.  Method for separating two or more dicarboxylic acids or fractions of two or more dicarboxylic acids from biological and/or chemical reaction solutions by adsorption, at a pH of 1 to 7 in a temperature range of 20°C-80°C, on at least partially non-polar hydrophobic adsorbents,
    from which an adsorbate is subsequently desorbed,
    **characterized in that**
    the adsorbents comprise highly crosslinked polymers (HCP) selected from polymethylenebiphenyl or polymethylenebenzene, wherein separation of two or more coexisting dicarboxylic acids is effected by selective adsorption, wherein the dicarboxylic acids are selected from maleic acid, itaconic acid, mesaconic acid, citraconic acid, methylsuccinic acid, tartaric acid and malic acid, and the adsorbed dicarboxylic acids are obtained from the adsorbents by elution with water, methanol, ethanol or acetone.

2.  Method according to Claim 1 ,
    **characterized in that** the pore volumes of the adsorbents comprise a magnitude of 0.01 cm$^3$/g to 4.0 cm$^3$/g and the adsorbents comprise a surface area in the range of 100 m$^2$/g to 4000 m$^2$/g.

**3.** Method according to either of the preceding claims,
**characterized in that** the adsorption is effected at a pH range between 2 and 5.

**4.** Method according to any of the preceding claims, **characterized in that** the desorption is effected at a pH in the range of greater than or equal to 7.

**5.** Method according to any of the preceding claims,
**characterized in that** in equimolar solutions of dicarboxylic acids and glucose, less than 1 mmol of glucose is adsorbed per g of the adsorbents.

**Revendications**

**1.** Procédé permettant de séparer une pluralité d'acides dicarboxyliques ou des fractions d'une pluralité d'acides dicarboxyliques de solutions réactionnelles biologiques et/ou chimiques par adsorption à un pH de 1 à 7 dans une gamme de températures allant de 20°C à 80°C sur des adsorbants hydrophobes au moins partiellement non polaires desquels un adsorbat est ensuite désorbé,
**caractérisé en ce que**
les adsorbants comprennent des polymères fortement réticulés (HCP) qui sont choisis parmi le polyméthylbiphényle ou le polyméthylbenzène, la séparation d'une pluralité d'acides dicarboxyliques coexistants étant effectuée par adsorption sélective, les acides dicarboxyliques étant choisis parmi les acides maléique, itaconique, mésaconique, citaconique, méthylsuccinique, tartrique et malique, et les acides dicarboxyliques adsorbés étant obtenus à partir des adsorbants par élution avec de l'eau, du méthanol, de l'éthanol ou de l'acétone.

**2.** Procédé selon la revendication 1, **caractérisé en ce que** le volume de pores des adsorbants va de 0,01 cm$^3$/g et 4,0 cm$^3$/g et les adsorbants ont une surface de l'ordre de 100 m$^2$/g et 4000 m$^2$/g.

**3.** Procédé selon l'une des revendications précédentes, **caractérisé en ce que** l'adsorption est effectuée dans une gamme de pH comprise entre 2 et 5.

**4.** Procédé selon l'une des revendications précédentes, **caractérisé en ce que** la désorption est effectuée à un pH supérieur ou égal à 7.

**5.** Procédé selon l'une des revendications précédentes, **caractérisé en ce que** moins de 1 mmol de glucose par g d'absorbants est adsorbée dans des solutions équimolaires d'acides dicarboxyliques et de glucose.

Abbildung 1

Abbildung 2

14

Abbildung 3

Abbildung 4

Abbildung 5

Abbildung 6

Abbildung 7

Abbildung 8

Abbildung 9

Abbildung 10

Abbildung 11

Abbildung 12

Abbildung 13

EP 3 253 730 B1

Abbildung 14

Abbildung 15

EP 3 253 730 B1

Abbildung 16

EP 3 253 730 B1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 102011120632 A1
- EP 0377430 A1
- US 2697725 A
- WO 2013169447 A1

### In der Beschreibung aufgeführte Nicht-Patentliteratur

- **CHIA-YUAN C. LEE et al.** Adsorption of oxalic, malonic and succinic acids on activated carbon. *Journal of chemical and engineering data,* 01. April 1986, vol. 31 (2
- **CHALINE DTONI et al.** Selctive liquid phase adsorption of 5-hydroxymethylfurfural on nanopourous hyper-cross-linked Polymers. *ACS sustainable chemistry & engineering,* 19. August 2014, vol. 2 (10